# EUROPEAN PATENT SPECIFICATION

(11) **EP 3 248 541 B1**
(45) Date of publication and mention of the grant of the patent: **08.09.2021**
(21) Application number: 17170725.0
(22) Date of filing: 11.05.2017
(51) Int. Cl.: A61B 7/00, A61B 5/0205, A61B 5/024, A61B 7/04, A61B 5/24, A61B 5/08, A61B 5/11, A61B 5/145, A61B 5/1455, A61B 5/00

(54) **PHYSIOLOGICAL MONITORING DEVICE**
PHYSIOLOGISCHES ÜBERWACHUNGSGERÄT
DISPOSITIF DE SUIVI PHYSIOLOGIQUE

(30) Priority: 23.05.2016 IT UA20163678
(43) Date of publication of application: 29.11.2017
(73) Proprietor: BIOCUBICA S.r.L., 20133 Milan (IT)
(72) Inventor: SALITO, Caterina, 20154 Milan (IT); BOVIO, Dario, 28066 Galliate (Novara) (IT); UVA, Barbara, 1004 Lausanne (CH)
(74) Representative: Lunati & Mazzoni S.r.L.

(56) References cited:
- CN-A- 105 592 781
- JP-A- 2013 102 784
- US-A1- 2007 113 654
- US-A1- 2015 141 774
- US-B1- 6 415 033
- Bosch Sensortec: "BME280: Final data sheet BME280 Combined humidity and pressure sensor", , 26 October 2015 (2015-10-26), XP055340087, Retrieved from the Internet: URL:https://ae-bst.resource.bosch.com/medi a/_tech/media/datasheets/BST-BME280_DS001- 11.pdf [retrieved on 2017-01-30]
- Sensing And Control Honeywell: "TruStability Board Mount Pressure Sensors Datasheet - HSC Series", , 31 August 2014 (2014-08-31), pages 1-34, XP055747576, Retrieved from the Internet: URL:https://eu.mouser.com/datasheet/2/187/ honeywell-sensing-trustability-hsc-series- high-acc-708740.pdf [retrieved on 2020-11-06]

## Description

The present invention relates to a physiological monitoring device according to independent claim 1, preferred embodiments are defined by dependent claims 2-8.

In particular, a device according to the invention is suitable for detecting physiological parameters within the context of conventional daily activities, as well as medical and sports activities.

At the current state-of-the-art, it is known that medical devices for physiological monitoring are characterised in that their working is principally based on contact of the device concerned with the upper dermal layer of the body.

Examples in this sense include instruments for detecting various characteristic physiological parameters commonly found in the context of everyday life: a thermometer is a simple instrument suitable for measuring body temperature by means of contact of the skin with a metal bulb.

Another example of a common object used for measuring physiological parameters is a sphygmomanometer, otherwise known as a pressure meter, suitable for determining diastolic and systolic blood pressure.

Besides the aforesaid devices, at the current state-of-the-art, it is possible to find other instruments for measuring and/or recording other types of physiological parameters of interest.

A stethophone endoscope is a commonly used instrument, which allows to listen to both high frequency and low frequency vibrations, hence its use in listening to internal organs in general.

Glucometers are commonly used instruments suitable for blood tests, for recording glucose in the blood.

Moreover, bracelets or wristwatches have been marketed, especially recently, which are mainly suitable for monitoring cardiac activity.

Within the context of processing physiological parameters relating to muscular activity, both in the clinical and commercial field, a number of recording techniques have been developed including, for example, mechanomyography (MMG) and electromyography (EMG).

These techniques are used to obtain sounds and electric activity respectively developed by muscular contractions originating from the movement of the muscle fibres.

In detail, the EMG is based on the frequency processing of the signals from electrodes positioned on the body or inside the body by means of needles for observing the innermost muscle fibres. State-of-the-art physiological monitoring devices are described, for instance, in patent applications US-A-2015/141774, US-A2007/113654, US-B-6415033 and JP-A-2013102784.

The known art described presents a number of important disadvantages.

In particular, the described devices are only able to monitor one physiological characteristic parameter and, in most cases require special recording procedures, just think of the sphygmomanometer, which prevent immediate use during activities that engage the whole body, such as, for example, sport.

In view of the above, an additional important disadvantage in the context of the current art is that the instruments currently in use are not easy to carry, with the exception of bracelets for recording heartbeats.

A further disadvantage of the known art relating to certain parameters lies in the invasiveness of the instruments used: just consider the glucometer, which includes the pricking of a fingertip, or the recording of internal muscle fibres using needles for the EMG.

A further disadvantage lies in the complexity and difficulty in obtaining acceptable forecasts for some of the apparatus currently in use, such as, for example devices for EMG.

The aim of the latter is to record and sample the frequencies of the signals coming from the moving muscle fibres, but they are affected by disturbances, such as, for example noise at 50 Hz coming from interference along the electrical line or signals from the antagonist muscles, which need significant filtering components for the signals.

In conclusion, and with particular reference to the muscular apparatus, the most serious disadvantage identified in the aforesaid art is the difficulty and lack of accuracy within the field of healthcare and particularly on individuals whose muscle fascia are compromised by pathologies such as, for example Amyotrophic Lateral Sclerosis (commonly known as ALS).

In this situation, the technical task at the base of the present invention is to develop a physiological monitoring device capable of substantially overcoming the stated disadvantages.

In the context of said technical task, an important objective is to obtain simultaneous monitoring, in real time, of a number of physiological characteristic parameters.

A further objective is to develop a device that guarantees high sensitivity to the physiological parameters under monitoring.

Another important objective is to develop a device that is easy to carry and can be used widely both in the field of sport, for monitoring physical training sessions and in the field of healthcare, for those cases that include pathologies such as muscular pathologies.

One last, but no less important, objective is to develop a device that is not invasive and which can be applied to the body with no limitations.

The technical task and specified objectives are achieved by means of the technical solution defined by the appended claim 1. Examples of preferred embodiments are described in the dependent claims.

The characteristics and advantages of the invention are clarified below by the detailed description of preferred embodiments, with reference to the accompanying drawings, wherein:

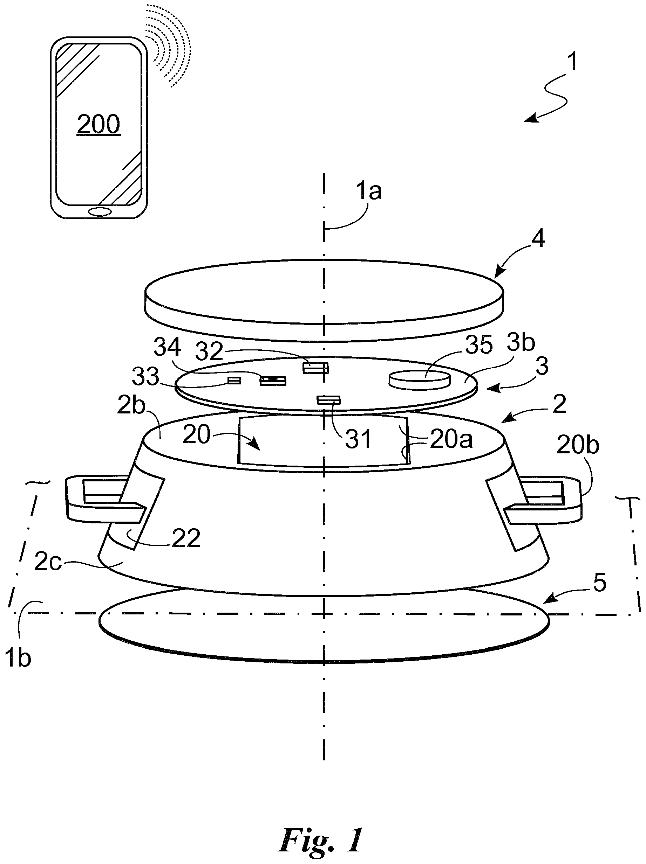

**Figure 1** shows a perspective view from the top of the device; and

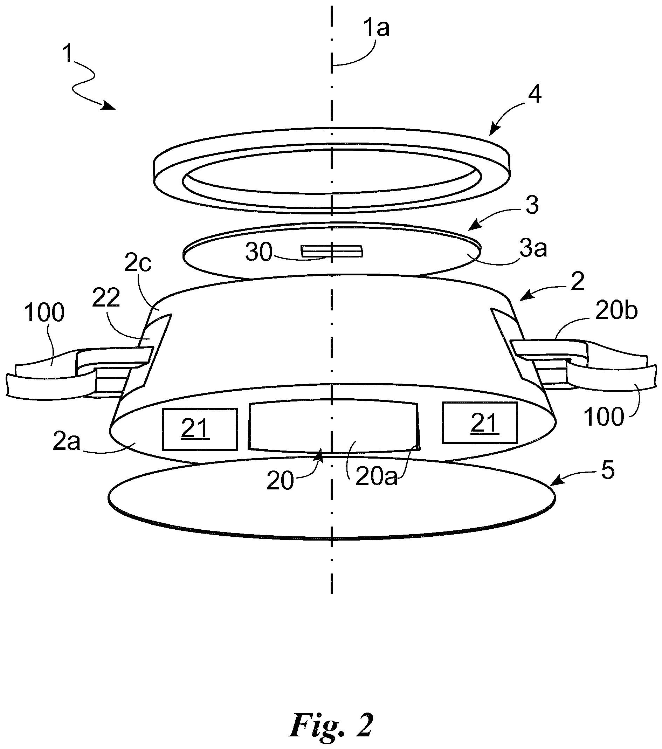

**Figure 2** illustrates a perspective view from the bottom of the device.

In this document, when measurements, values, shapes and geometrical references (such as perpendicularity and parallelism) are associated with words, such as "approximately" or other similar terms, for example "practically" or "substantially", they are to be understood as with the exception of measurement errors or inaccuracies resulting from production and/or manufacturing errors and, above all, with the exception of a slight divergence from the value, measurement, shape or geometrical reference with which it is associated. For example, if said terms are associated with a value, they preferably indicate a divergence of no more than 10% of the same value.

Furthermore, when terms such as "first", "second", "upper", "lower", "principal" and "secondary" are used, they do not necessarily identify an order, a relationship priority or relative position, but they may simply be used to distinguish different components more clearly.

With reference to the figures, the physiological monitoring device according to the invention is globally indicated with number **1**.

In brief, the inventive principle at the base of the physiological monitoring device 1 centres on the functional combination of the art known as mechanomyography and auscultation from a stethophone endoscope that is advantageously modified. Mechanomyography or MMG is a non-invasive technique, as stated in the introduction, able to acquire the sound produced by the muscular contractions originating from the movement of the muscle fibres: said sound of the muscle fibres is a signal whose frequency is comprised within a range of approximately 2-250 Hz. Said technique can be used in the clinical field, in athletics, for monitoring muscular fatigue or cybernetic as biofeedback for checking systems.

The stethophone endoscope is, as stated, a medical instrument, which allows the auscultation of the organs vibrating inside the body by means of sounds (pressure waves) received by the tympanum in the human ear through channelling tubes. Structurally, the physiological monitoring device 1 is preferably composed of four main components: a cup-shaped element **2,** defining a main axis **1a** and a reference plane **1b** perpendicular to the main axis 1a, a control element **3,** a cover **4** and, if necessary, a diaphragm **5.**

The cup-shaped element 2 is preferably made of a polymeric material and defines, for example, a lower base **2a** belonging to the reference plane 1b, centred on the main axis 1a, and an upper base **2b** with a variable geometry section, parallel to the plane 1b, smaller in relation to the projection of the lower base 2a on the reference plane 1b.

The upper base 2b is preferably surmounted by the control element 3.

The control element 3 is for example a plate, intended as a rigid element in which two sizes significantly predominate over the third, with variable geometry, comprising a lower surface **3a** parallel to the reference plane 1b and suitable for contact with the cup-shaped element 2, and an upper surface **3b** opposite the preceding one.

Furthermore, the cup-shaped element 2 defines a lateral surface 2c, where at least two slots **20b** are preferably positioned for possible connection with a little strap **100,** and comprising a through hole **20** that defines an internal surface **20a.**

The cup-shaped element 2 is substantially inspired by the aforesaid stethophone endoscope.

However, compared to this, the physiological monitoring device 1 presents advantageously different characteristics.

Recording of the physiological activities of interest takes place in the stethophone endoscope by acquisition of pressure waves, as stated previously, carried inside a tube, by the tympanum that is by a sound noise sensor.

Whereas, in the physiological monitoring device 1, the pressure signal is processed at the upper base 2b by a pressure sensor **30.** The latter is preferably comprised inside the control element 3 and in particular, in a position centred on the main axis 1a at the lower surface 3a.

The pressure sensor 30 is for example of the type BOSCH® BME 280 or Honeywell® HSCSRRN001NDAA3 and performs important functions in the context of the working of the monitoring device 1.

The monitoring device 1 is designed to transduce the pressure wave into an electric signal and subsequently digital and using a dedicated algorithm it enables the shifting of the low frequencies towards the high frequencies, making the otherwise inaudible pressure waves audible.

The pressure sensor 30 makes it possible to pick up and transmit signals at very low frequencies, lower than 5 Hz and preferably until null frequency or 0 Hz, which is usually not detectable with instruments belonging to the current state-of-the-art. In other words, the pressure sensor 30, unlike microphones, is capable of detecting and transmitting signals independently of the frequency, reaching far greater sensitivities.

Furthermore, the pressure sensor 30 enables body humidity and temperature to be recorded and, when resting on the abdomen, it is possible to measure the respiratory rate and transduce the sounds of opening and closing of the heart valves.

This is possible, principally because of a bidirectional valve **31** that allows the pressure to be controlled inside the cup-shaped element. This bidirectional valve 31 is also preferably comprised within the control element 3.

In particular, once closed, the valve 31 allows the device 1 to be sealed hermetically on the skin surface, closing the hole 20 again to facilitate the measurement of the respiratory rate. Whereas, when the valve 31 is open, it allows the offset to be removed, caused by overpressure that can form inside the hole 20 after the user has applied the instrument.

Thanks to the pressure sensor 30, the device 1 makes it possible to acquire defined frequency bands, but with reference to the cup-shaped element 2, by varying the lower base 2a, there is a contact surface, which allows the range of recordable frequencies to be modified.

Moreover, two optical sensors **21** are preferably placed on the lower base 2a, for example of the type SFH 7O60-OSRAM® or MAX30102 High-Sensitivity Pulse Oximeter and Heart-Rate sensor - MAXIM, symmetrically in relation to the main axis 1a.

These optical sensors 21 serve for pulsimetry, oxymetry and glucosimetry (Yadav et al., Signal Processing and Integrated Networks (SPIN), 2014 International Conference on. IEEE, 2014) and make it possible to monitor the coupling between the skin and the device.

Lastly, the cup-shaped element 2 preferably comprises force sensors **22** placed, for example, on the lateral surface 2c, in particular on at least one slot 20b.

The force sensors 22 are suitable for detecting, for example, the respiration frequency, measuring the change in tension on the slot caused by expansion of the user's body onto which the device 1 is applied.

Whereas, the control element 3 is also preferably fitted with electronic elements on its surface, in particular on the upper surface 3b: a micro-controller **32,** an IMU sensor **33,** a connection element **34** and a battery **35.**

In particular, the body humidity and temperature sensor 31 detects the user's temperature in the area below the cup-shaped element 2, obtaining data relating to the humidity, again on this area.

The micro-controller 32 is a common electronic element suitable for gathering and processing the measurement signals coming from the sensors on the control element 3.

The IMU 33 sensor, for example of the type BOSCH® BNO 055, comprises a gyroscope, magnetometer and accelerometer and is therefore suitable for calculating the acceleration, angular speed, angular position and ballistography.

The connection element 34 is, for example, a module of the type Bluetooth®, but it may also be of another type provided it guarantees the possibility to communicate with other external receiving devices **200** such as, for example, a smartphone or a PC or other.

Finally, the battery 35 serves to supply the whole device 1.

The aforesaid sensors can work independently, performing the functions for which they are designed, or they can work together in conjunction with the other sensors. For example, it is advantageously possible to combine the pressure sensor 30 with the IMU sensor 33 obtaining low frequency vibration measurements characterised by reduced uncertainty, making it possible to reinforce the mechanomyographical analysis. Or it is possible to combine the pressure sensor 30 data and optical sensor 21 data, or the IMU sensor 33 data and optical sensor 21 data to estimate blood pressure using pulse transit time (Wang et al. Int Conf Signal Process Proc. 2014 October; 2014: 115-118) or the pressure sensor 30 and force sensor 34 to estimate the respiratory rate.

The monitoring device 1 preferably also comprises a cover 4, for example made of a polymeric material, comprising a plate with variable geometry, of which at least one of the two surfaces is surmounted by a perimeter thickness 4c suitable for fully containing the control element 3 and suitable for being constrained, for example by gluing, to the cup-shaped element 2.

Finally, the physiological monitoring device 1 can comprise, if necessary, the diaphragm 5, as stated previously, on the surface of the base 2a.

Said diaphragm 5 can be of any shape and size and is preferably suitable for interfacing with the skin surface of the individual to be examined with damping effects on the lower frequencies and amplification effects of the higher frequencies. The working of the physiological monitoring device 1, previously described in substantially structural terms, is as follows.

It is preferably in contact with the skin surface, located above the muscular fascia concerned, and it is constrained to the limb, for example, by applying a little strap 100.

The physiological parameters of interest are subsequently recorded by the device 1 and sent to a receiver, such as for example a smartphone 200, to monitor the individual's physiological state, in real time, and in particular its muscular activity. The physiological monitoring device 1 according to the invention offers important advantages.

It is possible to monitor and control a number of physiological parameters simultaneously and synergistically using just the device 1 and without having to resort to complex operations to achieve said objectives.

The device 1 is also characterised by its outstanding sensitivity and transportability, as well as by its extensive versatility, which makes it simple and handy to use and transport.

In detail, the pressure sensor 30, which is characterised, as stated, by its surprisingly reduced bottom scale, advantageously guarantees greater sensitivity as it is able to record signals of considerably reduced intensity and low frequency close to those defined by the movement of the muscle fibres.

It makes it possible to assess signals, for example within ±1.8 mmHg and advantageously within frequencies ranging from 0 Hz to a few thousand, which are considerably reduced compared to the prior art and in particular to instruments like microphones, which use frequency response with a significantly higher scale (20 Hz-20 KHz).

In view of the above, the device 1 has a wide-ranging functionality that allows it to be used in fields such as sport and, equally important, health.

With particular reference to muscular fascia, the adoption of the mechanomyographical technique, together with the acquisition by a cup-shaped element 2, preferably a stethophone endoscope, with pressure sensors 30, enables the device 1 to be used and with considerable results, in terms of monitoring physiological activity, also in individuals affected by Amyotrophic Lateral Sclerosis, that is, having some compromised muscular fascia.

Finally, a further advantage lies in the fact that as the device 1 works in contact with the skin surface, it is not necessary to use invasive instruments.

In conclusion, the physiological monitoring device 1 according to the invention synergistically manages to combine elements aimed at improving the functionality and precision of the instrument.

Each sensor contributes favourably to increasing the accuracy of the data acquired from the other sensors and the same cup-shaped element 2 facilitates the acquisition of the pressure sensor 30 via the cavity or hole 20 inside it.

Moreover, the device 1 makes it possible to achieve accurate analyses for example on users affected by muscular dysfunctions without any logistic limitation, the device 1 being extremely compact and easily adapted to any position and configuration. All of these advantageous characteristics are, however, obtained from elements that are easy to find and consequently the physiological monitoring device 1 does not present any complications on a technological and financial level.

The invention is subject to variations that lie within the scope of the inventive concept defined by the claims.

For example, the cup-shaped element 2 can be round or oval or any other shape respecting and preserving the functionalities and previously expressed advantages. In said context, all of the details can be replaced by equivalent components and materials and they can be any shape or size.

## Claims

1. A physiological monitoring device (1) comprising:
- a cup-shaped element (2) suitable for acquiring signals from a skin surface,
- a control element (3) suitable for communicating with said cup-shaped element (2),
- said cup-shaped element (2) being suitable for acquiring signals comprising pressure pulsations,
- said control element (3) comprising a pressure sensor (30) suitable for detecting said signals independently of the frequency and with frequencies having a minimum detectable value that is at least lower than 5 Hz until a null frequency, and wherein said monitoring device (1) is designed to transduce the pressure wave into an electric signal, subsequently convert the electric signal to a digital signal, and to use a dedicated algorithm, said algorithm enabling the shifting of the low frequencies towards the high frequencies, making the otherwise inaudible pressure waves audible.

2. The physiological monitoring device (1) according to claim 1, wherein said pressure sensor (30) comprises a bottom-scale of ±1.8 mmHg

3. The physiological monitoring device (1) according to claim 1, wherein said cup-shaped element (2) comprises a stethophone endoscope.

4. The physiological monitoring device (1) according to one or more of the previous claims, wherein said cup-shaped element (2) comprises at least one optical sensor (21) and a force sensor (22).

5. The physiological monitoring device (1) according to one or more of the previous claims, wherein said pressure sensor (30) comprises the measurement of body temperature and humidity.

6. The physiological monitoring device (1) according to one or more of the previous claims, wherein said control element (3) comprises at least:
- a bidirectional valve (31),
- a micro-controller (32),
- an IMU sensor (33),
- a connection element (34), and
- a battery (35).

7. The physiological monitoring device (1) according to one or more of the previous claims, wherein said connection element (34) consists of a module chosen from a Bluetooth, Wi-Fi and wireless module suitable for communication with external elements.

8. The physiological monitoring device (1) according to claim 1, wherein said cup-shaped element (2) is connected to at least one diaphragm (5).

## Patentansprüche

1. Physiologisches Überwachungsgerät (1), umfassend:
- ein Schalenelement (2), das für die Erfassung von von einer Hautoberfläche kommenden Signalen geeignet ist,
- ein Steuerelement (3), das geeignet ist, mit dem genannten Schalenelement (2) zu kommunizieren,
- wobei das genannte Schalenelement (2) für die Erfassung von Signalen geeignet ist, die Druckpulsationen umfassen,
- wobei das genannte Steuerelement (3) einen Drucksensor (30) umfasst, der geeignet ist, die genannten Signale unabhängig von der Frequenz und mit Frequenzen mit einem messbaren Mindestwert von mindestens unter 5 Hz bis zu Nullfrequenz zu erfassen, und
- wobei das genannte Überwachungsgerät (1) dazu vorgesehen ist, die Druckwelle in ein elektrisches und dann digitales Signal umzuwandeln und über einen dedizierten Algorithmus das Verlagern der niedrigen Frequenzen zu hohen gestattet, um Druckwellen hörbar zu machen, die nicht hörbar sind.

2. Physiologisches Überwachungsgerät (1) nach dem vorangegangenen Anspruch, worin der genannte Drucksensor (30) einen Skalenendwert von ±1,8 mmHg umfasst.

3. Physiologisches Überwachungsgerät (1) nach Anspruch 1, worin das genannte Schalenelement (2) ein Stethophonendoskop umfasst.

4. Physiologisches Überwachungsgerät (1) nach einem oder mehreren der vorangegangenen Ansprüche, worin das genannte Schalenelement (2) mindestens einen optischen Sensor (21) und einen Kraftsensor (22) umfasst.

5. Physiologisches Überwachungsgerät (1) nach einem oder mehreren der vorangegangenen Ansprüche, worin der genannte Drucksensor (30) die Messung der Körpertemperatur und -feuchtigkeit umfasst.

6. Physiologisches Überwachungsgerät (1) nach einem oder mehreren der vorangegangenen Ansprüche, worin das genannte Steuerelement (3) mindestens Folgendes umfasst.
- ein bidirektionales Ventil (31),
- ein Mikrosteuergerät (32),
- einen IMU-Sensor (33),
- ein Verbindungselement (34), und
- eine Batterie (35).

7. Physiologisches Überwachungsgerät (1) nach einem oder mehreren der vorangegangenen Ansprüche, worin das genannte Verbindungselement (34) nach Wahl aus einem Bluetooth-, Wi-Fi- oder drahtlosen Modul besteht, das für die Kommunikation mit externen Elementen geeignet ist.

8. Physiologisches Überwachungsgerät (1) nach Anspruch 1, worin das genannte Schalenelement (2) mindestens mit einer Membran verbunden ist.

## Revendications

1. Dispositif de suivi physiologique (1) comprenant :
- un élément en coupe (2) destiné à l'acquisition de signaux provenant d'une surface cutanée,
- un élément de commande (3) destiné à communiquer avec ledit élément en coupe (2),
- ledit élément en coupe (2) est destiné à l'acquisition de signaux comprenant des pulsations de pression,
- ledit élément de commande (3) comprend un capteur de pression (30) destiné à détecter lesdits signaux indépendamment de la fréquence et avec des fréquences ayant une valeur minimale détectable au moins inférieure à 5 Hz jusqu'à une fréquence nulle, et
- où ledit dispositif de suivi (1) est affecté à traduire l'onde de pression en signal électrique et donc numérique, et à travers un algorithme dédié permet le changement des basses fréquences vers les hautes pour rendre audible des ondes de pression qui ne le sont pas.

2. Dispositif de suivi physiologique (1) selon la revendication précédente, où ledit capteur de pression (30) comprend un fond d'échelle de ±1,8 mmHg.

3. Dispositif de suivi physiologique (1) selon la revendication 1, où ledit élément en coupe (2) comprend un stéthophonendoscope.

4. Dispositif de suivi physiologique (1) selon l'une ou plusieurs des revendications précédentes, où ledit élément en coupe (2) comprend au moins un capteur optique (21) et un capteur de force (22).

5. Dispositif de suivi physiologique (1) selon l'une ou plusieurs des revendications précédentes, où ledit capteur de pression (30) comprend la mesure de la température et de l'humidité du corps.

6. Dispositif de suivi physiologique (1) selon l'une ou plusieurs des revendications précédentes, où ledit élément de commande (3) comprend au moins :
- une soupape bidirectionnelle (31),
- un microcontrôleur (32),
- un capteur IMU (33),
- un élément de liaison (34), et
- une batterie (35).

7. Dispositif de suivi physiologique (1) selon l'une ou plusieurs des revendications précédentes, où ledit élément de liaison (34) est constitué d'un module au choix entre Bluetooth, Wi-Fi et sans-fil destiné à la communication avec des éléments externes.

8. Dispositif de suivi physiologique (1) selon la revendication 1, où ladite coupe (2) est reliée à au moins un diaphragme (5).
